# EUROPÄISCHE PATENTSCHRIFT

(11) **EP 2 259 730 B1**
(45) Veröffentlichungstag und Bekanntmachung des Hinweises auf die Patenterteilung: **09.11.2011**
(21) Anmeldenummer: 08873817.4
(22) Anmeldetag: 21.11.2008
(51) Int. Cl.: A61B 17/122

(54) **LIGATURKLAMMERMAGAZIN**
LIGATURE CLAMP MAGAZINE
CASSETTE DE PINCES À LIGATURE

(30) Priorität: 10.04.2008 DE 102008018158
(43) Veröffentlichungstag der Anmeldung: 15.12.2010
(73) Patentinhaber: Aesculap AG, 78532 Tuttlingen (DE)
(72) Erfinder: WEISSHAUPT, Dieter, 78194 Immendingen (DE); DISCH, Alexander, 79104 Freiburg (DE)
(74) Vertreter: Hoeger, Stellrecht & Partner Patentanwälte
(86) Internationale Anmeldenummer: PCT/EP2008/009848
(87) Internationale Veröffentlichungsnummer: WO 2009/124576

(56) Entgegenhaltungen:
- DE-A1- 10 105 235
- DE-A1-102006 001 344
- US-A- 4 961 499
- US-A1- 2002 046 961
- US-A1- 2006 124 485

## Beschreibung

Die Erfindung betrifft ein Ligaturklammermagazin mit einem Lagerkörper und mit mehreren darin gelagerten C-förmig ausgebildeten, zwei über einen Steg miteinander verbundene Schenkel aufweisenden Ligaturklammern, wobei der Lagerkörper mehrere Fächer zur Aufnahme jeweils einer Ligaturklammer umfasst, mit einer an die Kontur der Ligaturklammer angepassten Stützfläche in jedem Fach, an der jeweils eine Ligaturklammer mit ihrer Innenfläche anliegt, und mit aus einer Halteposition in eine Freigabeposition verschwenkbaren, am Lagerkörper angeordneten Haltegliedern, die in der Halteposition die Ligaturklammer in ihrem Fach fixieren und in der Freigabeposition von der Ligaturklammer entfernt sind.

Ein derartiges Ligaturklammermagazin ist beispielsweise aus der EP 0 494 243 B1 bekannt. In einem solchen Magazin können mehrere Ligaturklammern nebeneinander aufbewahrt und mittels eines Entnahmewerkzeuges entnommen werden. Die Ligaturklammern sind in den Fächern auf einer Stützfläche aufliegend aufbewahrt und werden durch an die Außenseiten der Schenkel angedrückte Halteglieder in dieser Position fixiert. Allerdings erfolgt diese Fixierung ausschließlich durch einen Reibschluss, so dass die Gefahr besteht, dass sich eine Ligaturklammer unbeabsichtigt von der Stützfläche löst und dann eine undefinierte Lage in dem Fach des Lagerkörpers einnimmt. Dadurch besteht die Gefahr, dass eine Ligaturklammer von einem Entnahmewerkzeug nicht richtig erfasst werden kann, im schlimmsten Fall könnte eine Ligaturklammer auch aus dem Magazin herausfallen.

In der DE 101 05 235 A1 ist ein Ligaturklammermagazin und Ligaturklammer beschrieben gemäß dem Oberbegriff des Anspruchs 1, bei dem die Ligaturklammern durch Vorsprünge an dem Lagerkörper gehalten sind, die in Quernuten an der Außenseite der Ligaturklammern eingreifen.

Es ist Aufgabe der Erfindung, ein gattungsgemäßes Ligaturklammermagazin so auszubilden, dass die Ligaturklammern in ihm bis zur Entnahme besonders sicher fixiert werden.

Diese Aufgabe wird bei einem Ligaturklammermagazin der eingangs beschriebenen Art erfindungsgemäß dadurch gelöst, dass in den Schenkeln Durchbrechungen vorgesehen sind, in die die Halteglieder in der Halteposition eingreifen, und dass die Durchbrechung in dem Schenkel der Ligaturklammer ein längs der Schenkel verlaufender Schlitz ist.

Durch diese Ausgestaltung ergibt sich eine Festlegung der Ligaturklammern auf den Stützflächen durch einen Formschluss, in der Halteposition greifen die Halteglieder in die Durchbrechungen ein und stellen dadurch sicher, dass die Ligaturklammer sich aus der Lagerposition, in der sie auf der Stützfläche aufliegt, nicht unbeabsichtigt verschieben kann. Da die Durchbrechung in dem Schenkel der Ligaturklammer ein längs der Schenkel verlaufender Schlitz ist ergibt sich eine besonders sichere Fixierung der Ligaturklammer durch das Eintauchen der Halteglieder in die schlitzförmige Durchbrechung und den dabei eintretenden Formschluss.

Bei einer bevorzugten Ausführungsform ist vorgesehen, dass die Halteglieder an ihrer Oberseite eine Aufgleitfläche für ein Entnahmewerkzeug tragen, an der dieses beim Verschieben längs der Schenkel einer Ligaturklammer zur Anlage gelangt und dadurch die Halteglieder aus der Halteposition in die Freigabeposition verschwenkt. Allein durch das Einführen des Entnahmewerkzeuges wird somit die Freigabe der Ligaturklammern bewirkt, der Benutzer muss also für die Freigabe der Ligaturklammern keine besonderen Schritte vorsehen, es genügt das Anlegen des Entnahmewerkzeuges an die Außenseiten der Schenkel der Ligaturklammer.

Es kann insbesondere vorgesehen sein, dass die Halteglieder in ihrem in die Durchbrechung eingreifenden Bereich leistenförmig ausgebildet sind und mit ihren Seitenwänden an den seitlichen Rändern der Durchbrechung anliegen. Dadurch halten die Halteglieder die Ligaturklammern nicht nur in der Lagerposition, sie richten sie auch relativ zum Lagerkörper dadurch aus, dass die leistenförmigen Halteglieder in die schlitzförmigen Durchbrechungen eingreifen und mit ihren Seitenflächen an deren Seitenkanten anliegen. Dadurch ist eine exakte Justierung der Ligaturklammern im Lagerkörper sichergestellt.

Bei einer bevorzugten Ausführungsform kann vorgesehen werden, dass auch im Steg der Ligaturklammer eine Durchbrechung angeordnet ist, in die bei auf der Stützfläche aufliegender Ligaturklammer ein Vorsprung der Stützfläche hineinragt. Dieser Vorsprung dient ebenfalls der exakten Positionierung der Ligaturklammer auf der Stützfläche, er verhindert eine seitliche Verschiebung der Ligaturklammer auf der Stützfläche.

Auch bei dieser Durchbrechung im Steg der Ligaturklammer kann vorgesehen sein, dass diese Durchbrechung ein längs der Schenkel verlaufender Schlitz ist.

Auch der Vorsprung kann in diesem Falle leistenförmig ausgebildet sein und mit seinen Seitenwänden an den seitlichen Rändern der Durchbrechung im Steg der Ligaturklammer anliegen.

Besonders günstig ist es, wenn der Lagerkörper mit den Stützflächen und den Haltegliedern einstückig ausgebildet ist, insbesondere kann er aus einem vorzugsweise sterilisierbaren Kunststoff bestehen.

Dabei ist es vorteilhaft, wenn die Halteglieder über einen Biegesteg mit dem Lagerkörper verbunden sind, der beim Verschwenken der Halteglieder aus der Halteposition in die Freigabeposition dauerhaft plastisch verformt wird. Dadurch bleiben die Halteglieder nach dem Ausschwenken in die Freigabeposition in dieser Freigabeposition und bilden somit eine Anzeige dafür, dass die Ligaturklammer aus dem entsprechenden Fach, in dem die Halteglieder ausgeschwenkt sind, entnommen worden ist. Bei der Ausgestaltung des Lagerkörpers aus Kunststoff kann beispielsweise der Biegesteg so ausgebildet sein, dass es beim Ausschwenken der Halteglieder beim Material zum sogenannten "Weißbruch" kommt, also einer plastischen Verformung des Kunststoffmaterials, die bleibend ist.

Bei einer besonders bevorzugten Ausgestaltung ist vorgesehen, dass die Ligaturklammer zwei im Abstand zueinander verlaufende Längsstege aufweist, die an den Enden der Schenkel über einen Quersteg miteinander verbunden sind und die zwischen sich einen schlitzförmigen Zwischenraum definieren, der die Durchbrechungen in den Schenkeln und gegebenenfalls dem Steg der Ligaturklammer bildet, in die die Halteglieder beziehungsweise der Vorsprung der Stützfläche eingreifen. Derartige Ligaturklammern mit zwei parallel zueinander verlaufenden Längsstegen sind an sich bekannt, sie eignen sich besonders zur Verwendung bei einem Lagerkörper der beschriebenen Art, bei dem die Halteglieder in der Halteposition in den Zwischenraum zwischen den Längsstegen eingreifen und bei dem gegebenenfalls auch ein Vorsprung auf der Stützfläche in den Zwischenraum zwischen den Längsstegen eingreift.

Besonders vorteilhaft ist es, wenn die Durchbrechungen der Schenkel und die Durchbrechungen im Steg der Ligaturklammer durch weitere, die Längsstege der Ligaturklammer verbindende Querstege voneinander getrennt sind. Diese Querstege verleihen der Ligaturklammer eine zusätzliche Formstabilität und tragen dazu bei, dass die Ligaturklammern durch ein Entnahmewerkzeug besonders zuverlässig ergriffen werden können.

Es kann vorgesehen werden, dass die Halteglieder in ihrer Halteposition relativ zum Lagerkörper durch eine lösbare, elastische Rastverbindung fixiert sind. Dadurch wird sichergestellt, dass die Halteglieder nicht unbeabsichtigt aus ihrer Halteposition in die Freigabeposition verschwenkt werden können, eine Verschwenkung ist nur möglich, wenn die Halteglieder durch Einführen des Entnahmewerkzeuges mit einer definierten Kraft verschwenkt werden, welche die elastische Rastverbindung löst.

Zu diesem Zweck kann das Halteglied beispielsweise mindestens einen seitlichen Rastvorsprung tragen, der in eine Rastausnehmung des Lagerkörpers eingreift.

Vorzugsweise ist diese Lagerausnehmung in einer zwei nebeneinander liegende Fächer des Lagerkörpers trennenden Trennwand angeordnet. Diese Trennwände haben weiterhin den Vorteil, dass das Entnahmewerkzeug beim Einführen in ein Fach seitlich geführt wird.

Bei einer ersten bevorzugten Ausführungsform ist vorgesehen, dass die Halteglieder an der Außenseite der Ligaturklammer angeordnet sind, dass sie in ihrer Halteposition in Richtung auf die Mitte der Ligaturklammer eingeschwenkt sind und von außen nach innen in die Durchbrechung der Schenkel eintauchen, während sie in der Freigabestellung nach außen von der Ligaturklammer weg verschwenkt sind.

Es ist aber auch eine abgewandelte Ausgestaltung möglich, bei der vorgesehen ist, dass die Halteglieder an der Innenseite der Ligaturklammer angeordnet sind, dass sie in ihrer Halteposition in Richtung von der Mitte der Ligaturklammer weg ausgeschwenkt sind und von innen nach außen in die Durchbrechung der Schenkel eintauchen, während sie in der Freigabestellung nach innen zur Mitte der Ligaturklammer hin eingeschwenkt sind.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines Ligaturklammermagazins mit fünf darin eingesetzten Ligaturklammern und einer aus einem Fach des Lagerkörpers entnommenen Ligaturklammer;
- Figur 2:: eine perspektivische Ansicht des längs Linie 2-2 geschnittenen Ligaturklammermagazins der Figur 1 mit einer im vordersten Fach eingesetzten Ligaturklammer;
- Figur 3:: eine Ansicht ähnlich Figur 2 mit dem vordersten Fach ohne eingesetzte Ligaturklammer und mit einer separaten Ligaturklammer;
- Figur 4:: eine Schnittansicht des Ligaturklammermagazins der Figur 1 längs Linie 2-2 in Figur 1 mit zwei an eine Ligaturklammer angelegten Klemmarmen eines Entnahmewerkzeuges beim Beginn der Einführung der Klemmarme;
- Figur 5:: eine Ansicht ähnlich Figur 4 mit längs der Schenkel der Ligaturklammer vorgeschobenen Klemmarmen und ausgeschwenkten Haltegliedern;
- Figur 6:: eine Ansicht ähnlich Figur 5 mit vollständig ausgeschwenkten Haltegliedern und bei der Entnahme der Ligaturklammer mittels des Entnahmewerkzeuges;
- Figur 7:: eine perspektivische Ansicht des Ligaturklammermagazins der Figur 1 mit Ligaturklammern in zwei Fächern und mit vier nach der Entnahme der Ligaturklammern leeren Fächern;
- Figur 8:: eine perspektivische Ansicht eines weiteren bevorzugten Ausführungsbeispiels eines Ligaturklammermagazins mit zusätzliche Querstege aufweisenden Ligaturklammern;
- Figur 9:: eine perspektivische Ansicht des Ligaturklammermagazins der Figur 8 mit einem längs Linie 9-9 geschnittenen Lagerkörper;
- Figur 10:: eine perspektivische Ansicht des Ligaturklammermagazins der Figur 8 mit einem längs Linie 10-10 geschnittenen Lagerkörper;
- Figur 11:: eine perspektivische Ansicht eines weiteren bevorzugten Ausführungsbeispiels eines Ligaturklammermagazins mit von der Halteposition in die Freigabeposition nach innen einschwenkbaren Haltegliedern;
- Figur 12:: eine Schnittansicht des Ligaturklammermagazins der Figur 11 längs Linie 12-12 mit den Haltegliedern in der Halteposition und vor dem Anlegen eines Entnahmewerkzeuges und
- Figur 13:: eine Ansicht ähnlich Figur 12 mit an der Außenseite der Ligaturklammer angelegtem Entnahmewerkzeug und in die Freigabestellung nach innen eingeschwenkten Haltegliedern.

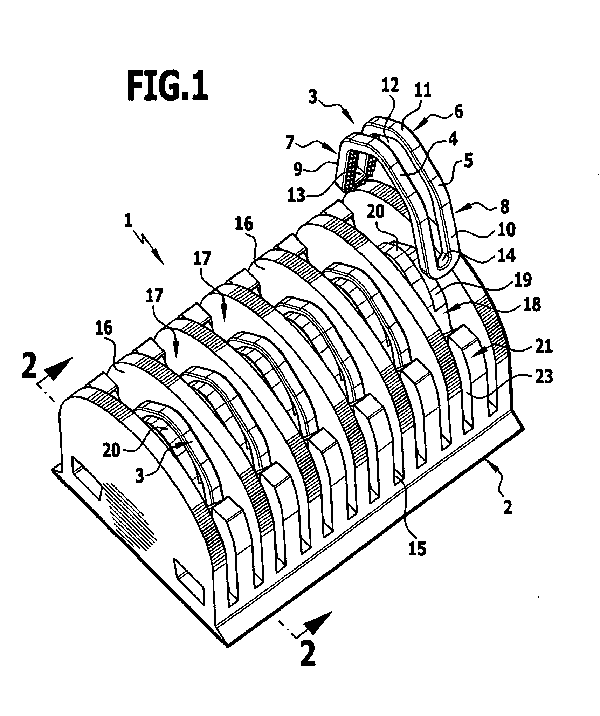

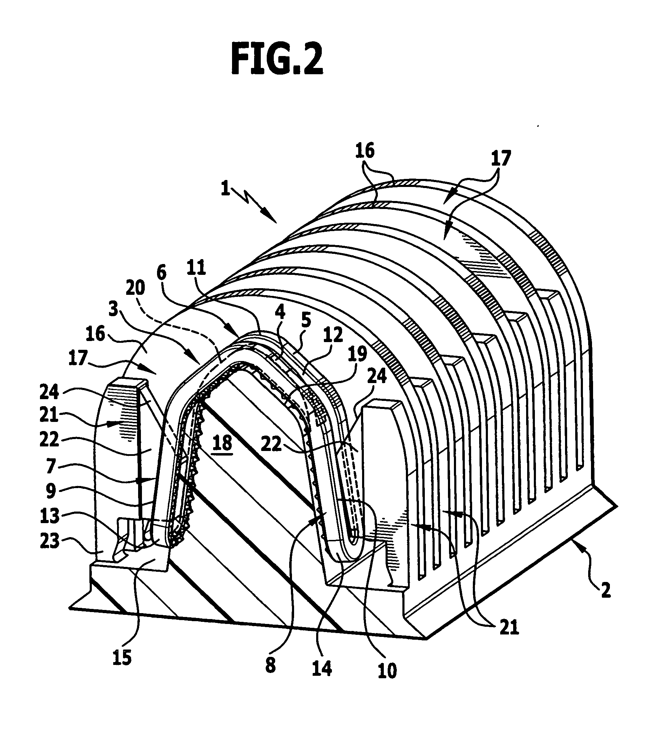

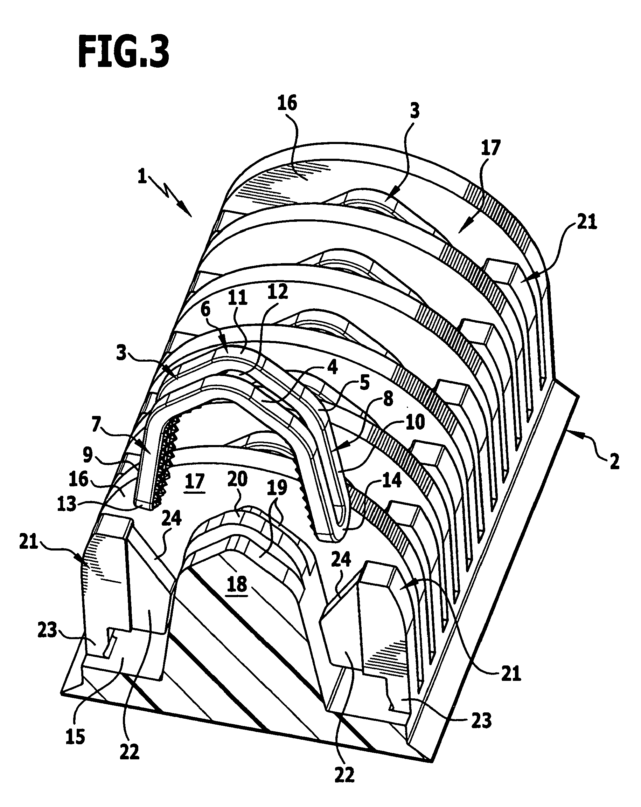

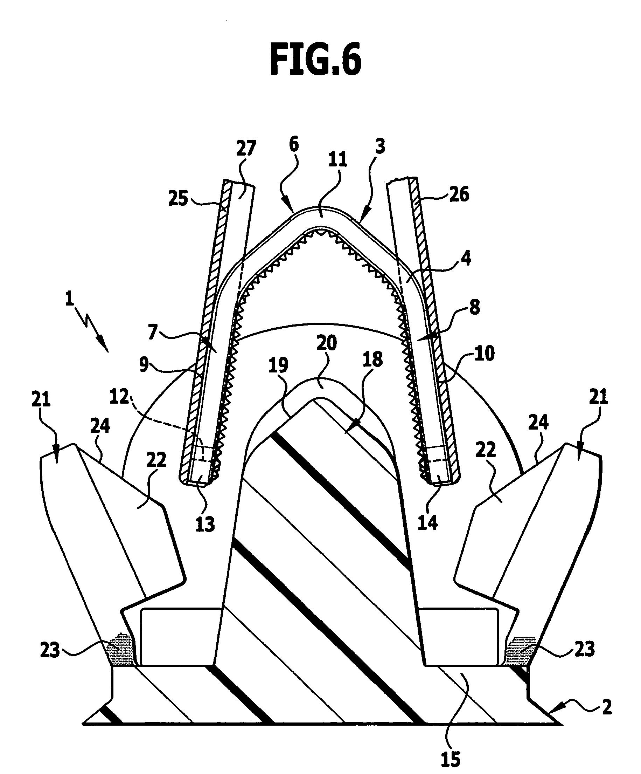

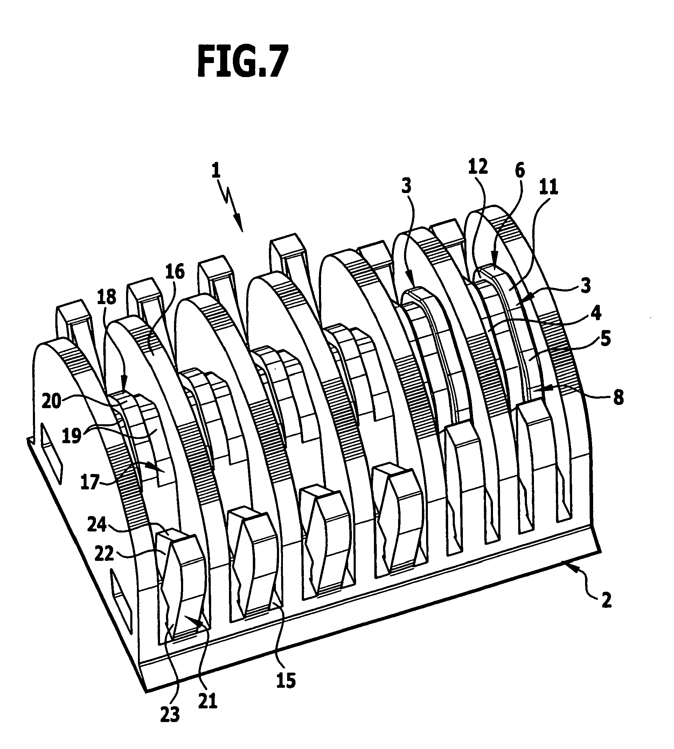

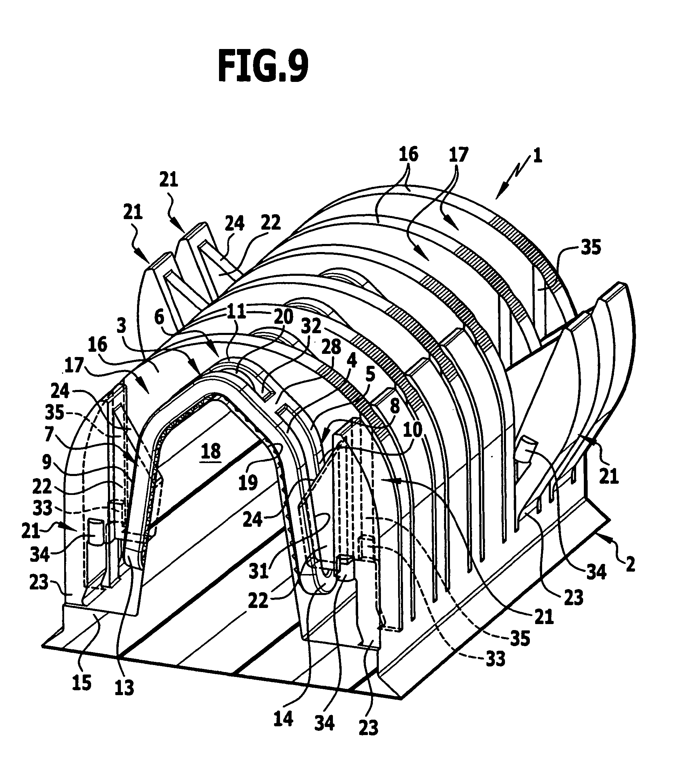

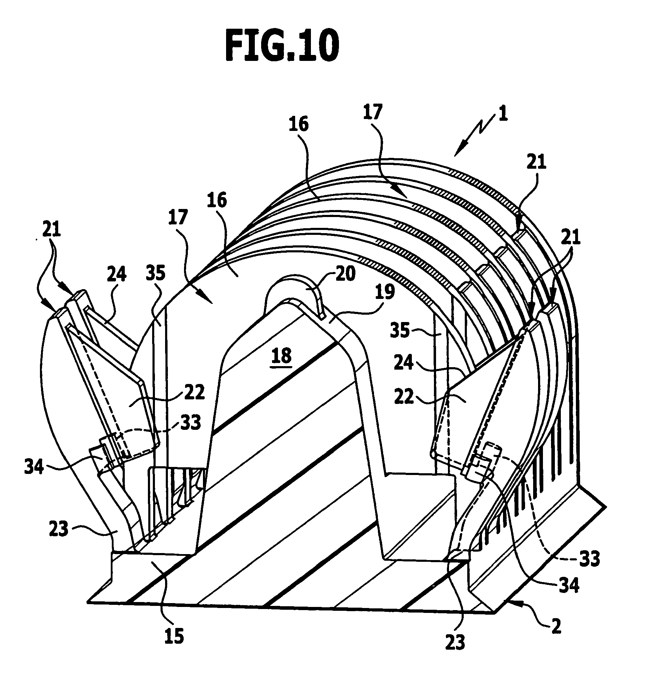

Das in den Figuren 1 bis 10 dargestellte Ligaturklammermagazin 1 umfasst einen Lagerkörper 2, in den eine Anzahl von Ligaturklammern 3 eingesetzt ist. Die Ligaturklammer 3 ist im wesentlichen C-förmig ausgebildet mit zwei über einen Steg 6 miteinander verbundenen Schenkeln 7, 8. Bei dem dargestellten Ausführungsbeispiel sind die Schenkel 7, 8 im wesentlichen eben ausgebildet und divergieren zu ihren freien Enden hin ein wenig, der Steg 6 dagegen ist V-förmig gebildet mit zwei ebenen Abschnitten 9, 10 und einer scharfen Abbiegung 11 zwischen den ebenen Abschnitten 9, 10.

Über den größten Teil der Länge der Schenkel 7, 8 und über den gesamten Steg 6 verläuft dabei in der Ligaturklammer 3 eine schlitzförmige Durchbrechung 12, so dass die Ligaturklammer 3 auch angesehen werden kann als eine Klammer, die aus zwei parallel zueinander verlaufenden Längsstegen 4, 5 besteht, die an den freien Enden der Schenkel 7, 8 in Form von abgebogenen Querstegen 13, 14 miteinander verbunden sind.

Der Lagerkörper 2 weist eine rechteckige Grundplatte 15 auf, von der mehrere ebene, parallel zur Schmalseite der Grundplatte 15 verlaufende senkrechte Trennwände 16 abstehen, die an ihrer Oberseite halbkreisförmig begrenzt sind und die zwischen sich Fächer 17 abtrennen. In jedem derartigen Fach 17 ist von der Grundplatte 15 ausgehend ein sich zwischen den beiden Trennwänden

16 nach oben erstreckende Stützkörper 18 angeordnet, dessen Außenkontur im wesentlichen der Innenkontur einer Ligaturklammer 3 angepasst ist, so dass die Außenfläche des Stützkörpers 18 eine Stützfläche 19 ausbildet, auf die eine Ligaturklammer 3 so aufgelegt werden kann, dass sie mit ihrem Steg 6 und mit ihren Schenkeln 7 an dieser Stützfläche 19 anliegt.

Die Stützfläche 19 trägt an ihrem oberen Ende einen leistenförmigen Vorsprung 20, der im Bereich des Steges 6 in die Durchbrechung 12 eingreift, also in den Zwischenraum zwischen den zwei nebeneinander verlaufenden Längsstegen 4, 5 der Ligaturklammer 3, so dass eine auf die Stützfläche 19 aufgelegte Ligaturklammer 3 dadurch gegen eine seitliche Verschiebung gesichert ist. Der Vorsprung 20 ist vorzugsweise so breit wie der Zwischenraum zwischen den Längsstegen 4, 5, so dass durch das Eingreifen des leistenförmigen Vorsprunges 20 in die Durchbrechung 12 auch eine Ausrichtung der Ligaturklammer 3 auf dem Stützkörper 18 erfolgt.

In jedem Fach 17 sind im Bereich des Längsrandes der Grundplatte 15 auf gegenüberliegenden Seiten des Faches 17 zwei Halteglieder 21 angeformt, die jeweils einen nach innen weisenden, leistenförmigen Vorsprung 22 tragen, der im Bereich der Schenkel 7, 8 in die Durchbrechung 12 einer auf die Stützfläche 19 aufgelegten Ligaturklammer 3 eingreift. Die Abmessungen sind dabei so gewählt, dass dieser leistenförmige Vorsprung 22 mit seinen Seitenflächen an den Seitenkanten der Durchbrechung 12 anliegt und damit ebenfalls zu einer Ausrichtung der Ligaturklammer 3 beiträgt, außerdem liegt dieser Vorsprung 22 an den Querstegen 13 beziehungsweise 14 der beiden Schenkel 7 an und verhindert somit, dass die Ligaturklammer 3 unbeabsichtigt von der Stützfläche 19 abgehoben werden kann.

Die Halteglieder 21 sind mit der Grundplatte 15 über einen Biegesteg 23 mit relativ kleinem Querschnitt verbunden und normalerweise so ausgebildet, dass sie mit ihrer Außenkontur mit der Außenkontur der Trennwände 16 fluchten und dabei mit dem Vorsprung 22 in die Durchbrechung 12 eingreifen. In dieser Stellung halten die Halteglieder 21 somit die Ligaturklammer 3 auf dem Stützkörper 18, diese Stellung der Halteglieder 21 wird daher als Halteposition bezeichnet.

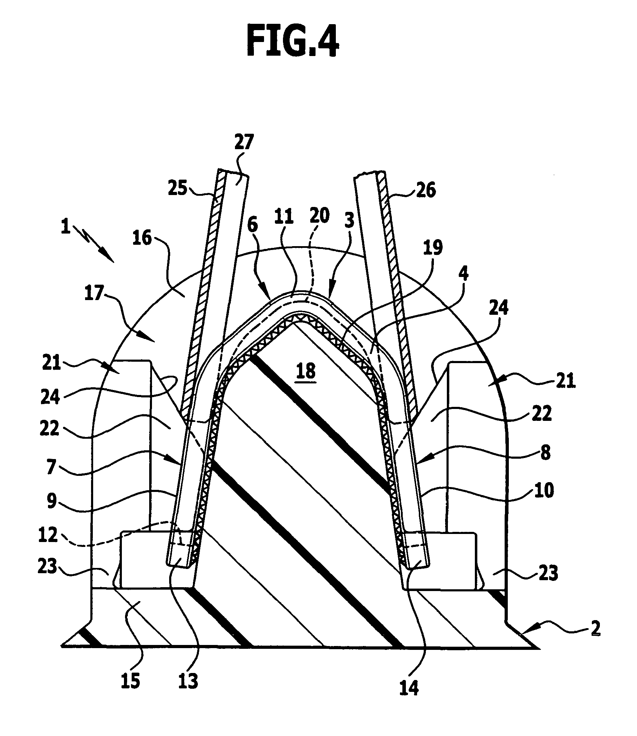

Auf der Oberseite weisen die Vorsprünge 22 eine von außen nach innen schräg abfallende Aufgleitfläche 24 auf, die in der Halteposition zumindest teilweise über die Ligaturklammer 3 seitlich hervorsteht, so dass in diesem Bereich Klemmbacken 25, 26 eines Entnahmewerkzeuges aufgesetzt werden können (Figur 4), wenn diese Klemmarme 25, 26 längs der Schenkel 7, 8 von oben her in ein Fach 17 eingeführt werden. Dabei legen sich die Klemmarme 25, 26 außenseitig an die Schenkel 7, 8 an und schwenken gleichzeitig beim weiteren Einschieben die Halteglieder 21 nach außen. Bei dieser Schwenkbewegung wird der Biegesteg 23 plastisch verformt, so dass diese Ausschwenkbewegung der Halteglieder irreversibel ist. Bei dem Ausschwenken treten die Vorsprünge 22 aus der Durchbrechung 12 heraus und geben dadurch die Ligaturklammer 3 frei.

Ergänzend wird darauf hingewiesen, dass durch die schrägen Aufgleitflächen 24 auch das Einführen der Klemmarme 25, 26 erleichtert wird, da die Aufgleitflächen 24 gleichzeitig eine zentrierende Funktion auf die beiden Klemmarme 25, 26 ausüben und diese in die richtige Anlageposition an den Schenkeln 7, 8 lenken.

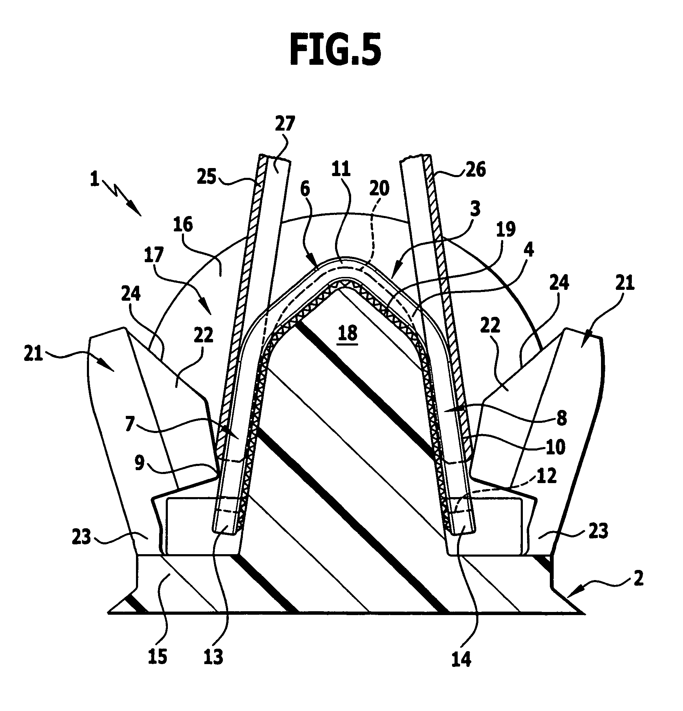

Die Klemmarme 25, 26 tragen an ihrer Innenseite einen leistenförmigen Vorsprung 27, der beim Vorschieben der Klemmarme 25, 26 an den Schenkeln 7, 8 in die Durchbrechung 12 eintritt und somit die Ligaturklammer 3 relativ zu den Klemmarmen 25, 26 ausrichtet (Figuren 4 und 5).

Sobald die Klemmarme 25, 26 vollständig in das Fach 17 eingeschoben sind, liegen sie vollflächig an der Außenseite der Schenkel 7, 8 an und haben die Halteglieder 21 vollständig nach außen geschwenkt. Durch die flächige Anlage wird die Ligaturklammer 3 jetzt im Reibschluss an den Klemmarmen 25, 26 gehalten, jedoch nicht mehr durch die Halteglieder 21 relativ zum Lagerkörper 2 fixiert. Daher kann beim Herausziehen der Klemmarme 25, 26 aus dem Fach 17 die zwischen den Klemmarmen 25, 26 eingeklemmte Ligaturklammer 3 aus dem Fach 17 entnommen und mittels des Entnahmewerkzeuges an den Einsatzort transferiert werden.

Nach der Entnahme einer Ligaturklammer 3 aus einem Fach bleiben die Halteglieder 21 dieses Faches in der ausgeschwenkten Stellung stehen, in der sie seitlich über die durch die Trennwände 16 definierte Außenkontur des Lagerkörpers 2 hervorstehen, damit wird für den Benutzer angezeigt, dass aus diesem Fach die Ligaturklammer 3 bereits entnommen worden ist.

Während die Ligaturklammern 3 üblicherweise aus einem körperverträglichen Metall bestehen, beispielsweise aus Titan oder einer Titanlegierung, ist es vorteilhaft, den Lagerkörper 2 einstückig aus einem vorzugsweise sterilisierbaren Kunststoff herzustellen, beispielsweise als Spritzteil. Dabei können an dem Lagerkörper 2 sowohl die Stützkörper 18 als auch die Halteglieder 21 einstückig angespritzt sein, dasselbe gilt hinsichtlich der Trennwände 16.

Bei dem Ausführungsbeispiel der Figuren 1 bis 7 werden Ligaturklammern 3 verwendet, die zwischen den Längsstegen 4, 5 eine einzige, durchgehende, schlitzförmige Durchbrechung 12 aufweisen.

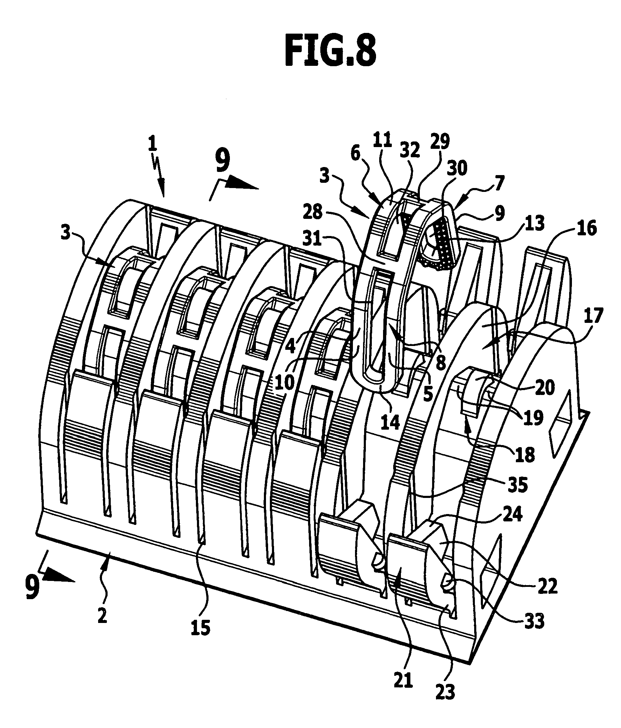

Im Gegensatz dazu werden bei dem Ausführungsbeispiel der Figuren 8 bis 10, bei denen einander entsprechende Teile dieselben Bezugszeichen tragen, Ligaturklammern 3 verwendet, bei denen die Längsstege 4, 5 durch zusätzliche Querstege 28, 29 miteinander verbunden sind. Dadurch ergeben sich im Bereich der Schenkel 7, 8 einerseits und im Bereich des Steges 6 andererseits voneinander getrennte Durchbrechungen 30, 31 beziehungsweise 32. Durch diese Querstege 28, 29 wird die Formstabilität der Ligaturklammer 3 erhöht, außerdem tragen diese Querstege dazu bei, dass die Klemmarme 25, 26 derartige Ligaturklammern noch zuverlässiger durch einen Klemmsitz greifen können.

Entsprechend der geringeren Länge der Durchbrechung 32 im Steg 6 sind auch die Vorsprünge 20 auf der Stützfläche 19 beim Ausführungsbeispiel der Figuren 8 bis 10 kürzer ausgebildet als bei dem Ausführungsbeispiel der Figuren 1 bis 7.

Bei dem Ausführungsbeispiel der Figuren 8 bis 10 ist außerdem vorgesehen, dass die Halteglieder 21 an ihren Seitenflächen seitlich abstehende Rastvorsprünge 33, 34 tragen, die bei in die Halteposition eingeschwenkten Haltegliedern 21 in nutenförmige Vertiefungen 35 der benachbarten Trennwände 16 eingreifen und somit eine lösbare, elastische Rastverbindung ausbilden, durch die die Halteglieder 21 in ihrer Halteposition festgelegt werden. Dies erhöht die Zuverlässigkeit der Fixierung der Ligaturklammern 3 im Lagerkörper 2, diese Rastverbindung kann nur dadurch gelöst werden, dass mittels der Klemmarme 25, 26 die Halteglieder 21 ausgeschwenkt werden.

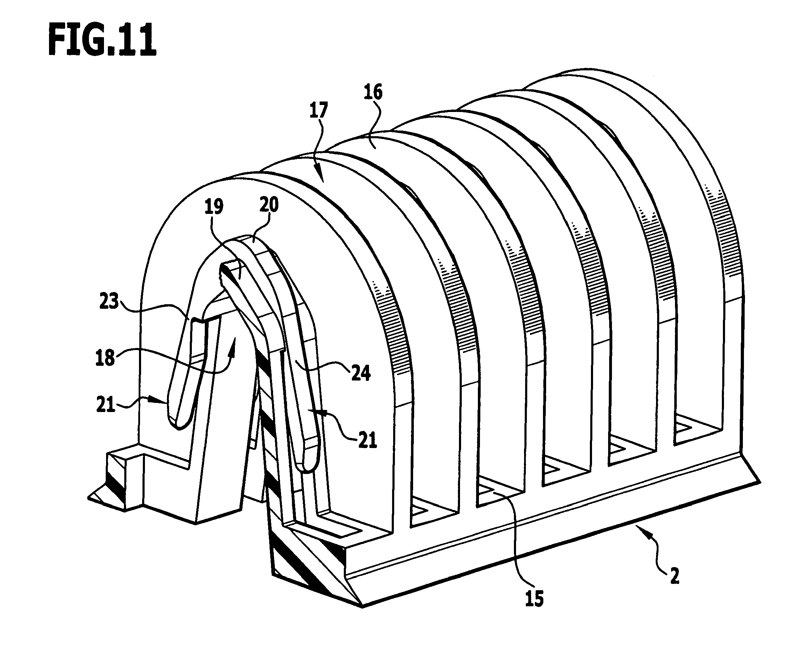

Das Ausführungsbeispiel der Figuren 11 bis 13 ist ähnlich aufgebaut wie die Ausführungsbeispiele der Figuren 1 bis 10, einander entsprechende Teile tragen daher dieselben Bezugszeichen.

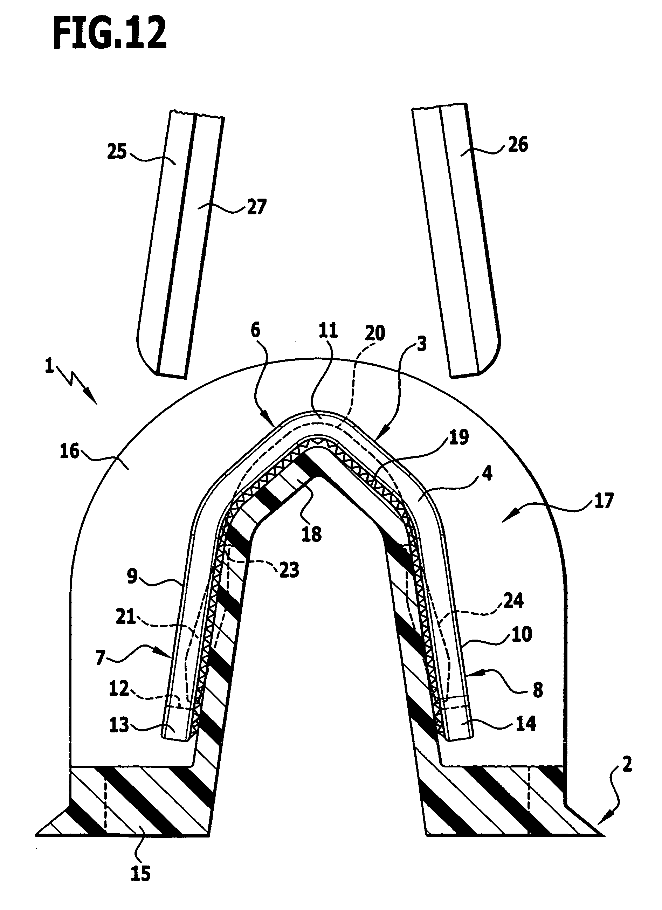

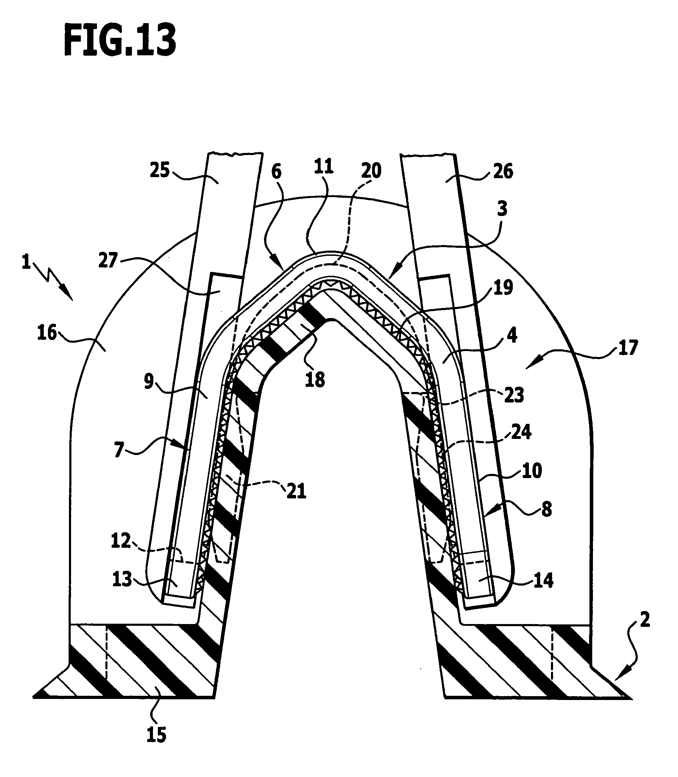

Während bei dem Ausführungsbeispiel der Figuren 1 bis 10 die Halteglieder 21 an der Außenseite der Ligaturklammer 3 angeordnet sind und in ihrer Halteposition mit dem leistenförmigen Vorsprung 22 von außen nach innen in die schlitzförmige Durchbrechung 12 der Ligaturklammer 3 eintauchen, ist bei dem Ausführungsbeispiel der Figuren 11 bis 13 die Anordnung der Halteglieder 22 auf die Innenseite der Ligaturklammer 3 verlagert, das heißt die Halteglieder 22 sind unmittelbar anschließend an die Stützfläche 19 so angeordnet, dass sie nur an ihrem oberen Ende über einen Biegesteg 23 mit der Stützfläche 19 verbunden sind, während sie in ihrem unteren Ende frei enden. In der Halteposition (Figur 12) sind die Halteglieder 21 gegenüber der Stützfläche 19 nach außen verschwenkt und tauchen dadurch von innen nach außen in die schlitzförmige Durchbrechung 12 der Ligaturklammer 3 ein. Beim Anlegen der Klemmarme 25, 26 des Entnahmewerkzeuges an die Außenseite der Ligaturklammer 3 gleiten diese Klemmarme 25, 26 an der Außenseite der in die Halteposition ausgeschwenkten Halteglieder 21 entlang und verschwenken die Halteglieder 21 dadurch nach innen, bis sie aus den schlitzförmigen Durchbrechungen 12 austreten und dadurch die Ligaturklammer 3 freigeben (Figur 13). Die Außenseiten der Halteglieder 21 bilden somit ebenfalls eine schräg abfallende Aufgleitfläche 24 aus, die jedoch in diesem Fall von oben nach unten von innen schräg nach außen verläuft. Nach dieser Freigabe der Ligaturklammer 3 kann diese mittels des Entnahmewerkzeuges aus dem Magazin entnommen werden.

Die anhand der unterschiedlichen Ausführungsbeispiele beschriebenen Ausgestaltungen der Ligaturklammern einerseits und der Lagerkörper andererseits können auch untereinander vertauscht und in unterschiedlichen Kombinationen an einem Ligaturklammermagazin verwirklicht werden.

## Patentansprüche

1. Ligaturklammermagazin mit einem Lagerkörper (2) und mit mehreren darin gelagerten C-förmig ausgebildeten, zwei über einen Steg (6) miteinander verbundene Schenkel (7, 8) aufweisenden Ligaturklammern (3), wobei der Lagerkörper (2) mehrere Fächer (17) zur Aufnahme jeweils einer Ligaturklammer (3) umfasst, mit einer an die Kontur der Ligaturklammer (3) angepassten Stützfläche (19) in jedem Fach (17), an der jeweils eine Ligaturklammer (3) mit ihrer Innenfläche anliegt, mit aus einer Halteposition in eine Freigabeposition verschwenkbaren, am Lagerkörper (2) angeordneten Haltegliedern (21), die in der Halteposition die Ligaturklammer (3) in ihrem Fach (17) fixieren und in der Freigabeposition von der Ligaturklammer (3) entfernt sind, **dadurch gekennzeichnet, dass** in den Schenkeln (7, 8) Durchbrechungen (12; 30, 31) vorgesehen sind, in die die Halteglieder (21) in der Halteposition eingreifen, und dass die Durchbrechung (12; 30, 31) in dem Schenkel (7, 8) der Ligaturklammer (3) ein längs der Schenkel (7, 8) verlaufender Schlitz ist.

2. Ligaturklammermagazin nach Anspruch 1, **dadurch gekennzeichnet, dass** die Halteglieder (21) an ihrer Oberseite eine Aufgleitfläche (24) für ein Entnahmewerkzeug (25, 26) tragen, an der dieses beim Vorschieben längs der Schenkel (7, 8) einer Ligaturklammer (3) zur Anlage gelangt und dadurch die Halteglieder (21) aus der Halteposition in die Freigabeposition verschwenkt.

3. Ligaturklammermagazin nach einem der voranstehenden Ansprüche, **dadurch gekennzeichnet, dass** die Halteglieder (21) in ihrem in die Durchbrechung (12; 30, 31) eingreifenden Bereich (22) leistenförmig ausgebildet sind und mit ihren Seitenwänden an den seitlichen Rändern der Durchbrechung (12; 30, 31) anliegen.

4. Ligaturklammermagazin nach einem der voranstehenden Ansprüche, **dadurch gekennzeichnet, dass** im Steg (6) der Ligaturklammer (3) eine Durchbrechung (12; 32) angeordnet ist, in die bei auf der Stützfläche (19) aufliegender Ligaturklammer (3) ein Vorsprung (20) der Stützfläche (19) hineinragt.

5. Ligaturklammermagazin nach Anspruch 4, **dadurch gekennzeichnet, dass** die Durchbrechung (12; 32) in dem Steg (6) der Ligaturklammer (3) ein längs des Steges (6) verlaufender Schlitz ist.

6. Ligaturklammermagazin nach Anspruch 5, **dadurch gekennzeichnet, dass** der Vorsprung (20) in seinem in die Durchbrechung (12; 32) eingreifenden Bereich leistenförmig ausgebildet ist und mit seinen Seitenwänden an den seitlichen Rändern der Durchbrechung (12; 32) anliegt.

7. Ligaturklammermagazin nach einem der voranstehenden Ansprüche, **dadurch gekennzeichnet, dass** der Lagerkörper (2) mit den Stützflächen (19) und den Haltegliedern (21) einstückig ausgebildet ist.

8. Ligaturklammermagazin nach einem der voranstehenden Ansprüche, **dadurch gekennzeichnet, dass** der Lagerkörper (2) mit den Stützflächen (19) und den Haltegliedern (21) aus Kunststoff besteht.

9. Ligaturklammermagazin nach Anspruch 8, **dadurch gekennzeichnet, dass** die Halteglieder (21) über einen Biegesteg (23) mit dem Lagerkörper (2) verbunden sind, der beim Verschwenken der Halteglieder (21) aus der Halteposition in die Freigabeposition dauerhaft plastisch verformt wird.

10. Ligaturklammermagazin nach einem der voranstehenden Ansprüche, **dadurch gekennzeichnet, dass** die Ligaturklammer (3) zwei im Abstand nebeneinander verlaufende Längsstege (4, 5) aufweist, die an den Enden der Schenkel (7, 8) über einen Quersteg (13, 14) miteinander verbunden sind und die zwischen sich einen schlitzförmigen Zwischenraum definieren, der die Durchbrechungen (12; 30, 31, 32) in den Schenkeln (7, 8) und gegebenenfalls dem Steg (6) der Ligaturklammer (3) bildet, in die die Halteglieder (21) beziehungsweise der Vorsprung (20) der Stützfläche (19) eingreifen.

11. Ligaturklammermagazin nach Anspruch 10, **dadurch gekennzeichnet, dass** die Durchbrechungen (30, 31) der Schenkel (7, 8) und die Durchbrechung (32) im Steg (6) der Ligaturklammer (3) durch die Längsstege (4, 5) der Ligaturklammer (3) verbindende Querstege (28, 29) voneinander getrennt sind.

12. Ligaturklammermagazin nach einem der voranstehenden Ansprüche, **dadurch gekennzeichnet, dass** die Halteglieder (21) in ihrer Halteposition relativ zum Lagerkörper (2) durch eine lösbare, elastische Rastverbindung (33, 34, 35) fixiert sind.

13. Ligaturklammermagazin nach Anspruch 12, **dadurch gekennzeichnet, dass** das Halteglied (21) mindestens einen seitlichen Rastvorsprung (33, 34) trägt, der in eine Rastausnehmung (35) des Lagerkörpers (2) eingreift.

14. Ligaturklammermagazin nach Anspruch 13, **dadurch gekennzeichnet, dass** die Lagerausnehmung (35) in einer zwei nebeneinander liegende Fächer (17) des Lagerkörpers (2) trennenden Trennwand (16) angeordnet ist.

15. Ligaturklammermagazin nach einem der voranstehenden Ansprüche, **dadurch gekennzeichnet, dass** die Halteglieder (21) an der Außenseite der Ligaturklammer (3) angeordnet sind, dass sie in ihrer Halteposition in Richtung auf die Mitte der Ligaturklammer (3) eingeschwenkt sind und von außen nach innen in die Durchbrechung (12; 30, 31) der Schenkel (7, 8) eintauchen, während sie in der Freigabestellung nach außen von der Ligaturklammer (3) weg verschwenkt sind.

16. Ligaturklammermagazin nach einem der Ansprüche 1 bis 14, **dadurch gekennzeichnet, dass** die Halteglieder (21) an der Innenseite der Ligaturklammer (3) angeordnet sind, dass sie in ihrer Halteposition in Richtung von der Mitte der Ligaturklammer (3) weg ausgeschwenkt sind und von innen nach außen in die Durchbrechung (12; 30, 31) der Schenkel (7, 8) eintauchen, während sie in der Freigabestellung nach innen zur Mitte der Ligaturklammer (3) hin eingeschwenkt sind.

## Claims

1. Surgical clip cartridge with a housing member (2) and with a plurality of C-shaped surgical clips (3) stored therein having two limbs (7, 8) connected to one another by a web (6), the housing member (2) having a plurality of compartments (17) each for receiving a surgical clip (3), with a support face (19) adapted to the contour of the surgical clip (3) in each compartment (17), against which support face a respective surgical clip (3) abuts with its inner face, with holding members (21) arranged on the housing member (2), which can be pivoted from a holding position into a release position and, in the holding position, fix the surgical clip (3) in its compartment (17) and, in the release position, are removed from the surgical clip (3), **characterised in that** openings (12; 30, 31) are provided in the limbs (7, 8), in which the holding members (21) engage in the holding position, and **in that** the opening (12; 30, 31) in the limb (7, 8) of the surgical clip (3) is a slot running along the limbs (7, 8).

2. Surgical clip cartridge according to claim 1, **characterised in that** the holding members (21) bear, on their upper side, a sliding face (24) for a removal tool (25, 26), with which sliding face said tool comes into contact when pushed forward along the limbs (7, 8) of a surgical clip (3) and thereby pivots the holding members (21) from the holding position into the release position.

3. Surgical clip cartridge according to either of the preceding claims, **characterised in that** the holding members (21) are in the form of strips in their region (22) engaging in the opening (12; 30, 31) and rest with their side walls against the lateral edges of the opening (12; 30, 31).

4. Surgical clip cartridge according to any one of the preceding claims, **characterised in that** arranged in the web (6) of the surgical clip (3) is an opening (12; 32), into which a projection (20) of the support face (19) projects when the surgical clip (3) abuts the support face (19).

5. Surgical clip cartridge according to claim 4, **characterised in that** the opening (12; 32) in the web (6) of the surgical clip (3) is a slot running along the web (6).

6. Surgical clip cartridge according to claim 5, **characterised in that** the projection (20) is formed as a strip in its region engaging in the opening (12; 32) and abuts with its side walls against the lateral edges of the opening (12; 32).

7. Surgical clip cartridge according to any one of the preceding claims, **characterised in that** the housing member (2) is configured in one piece with the support faces (19) and the holding members (21).

8. Surgical clip cartridge according to any one of the preceding claims, **characterised in that** the housing member (2) with the support faces (19) and the holding members (21) consists of plastics material.

9. Surgical clip cartridge according to claim 8, **characterised in that** the holding members (21) are connected by a bending web (23) to the housing member (2), which is permanently plastically deformed when the holding members (21) are pivoted out of the holding position into the release position.

10. Surgical clip cartridge according to any one of the preceding claims, **characterised in that** the surgical clip (3) has two longitudinal webs (4, 5) extending next to one another at a spacing, which are connected to one another at the ends of the limbs (7, 8) by a transverse web (13, 14) and, between them, define a slot-like space which forms the openings (12; 30, 31, 32) in the limbs (7, 8) and optionally in the web (6) of the surgical clip (3), in which the holding members (21) and/or the projection (20) of the support face (19) engage.

11. Surgical clip cartridge according to claim 10, **characterised in that** the openings (30, 31) of the limbs (7, 8) and the opening (32) in the web (6) of the surgical clip (3) are separated from one another by transverse webs (28, 29) connecting the longitudinal webs (4, 5) of the surgical clip (3).

12. Surgical clip cartridge according to any one of the preceding claims, **characterised in that** the holding members (21) are fixed in their holding position relative to the housing member (2) by a releasable, resilient latching connection (33, 34, 35).

13. Surgical clip cartridge according to claim 12, **characterised in that** the holding member (21) carries at least one lateral latching projection (33, 34) which engages in a latching recess (35) of the housing member (2).

14. Surgical clip cartridge according to claim 13, **characterised in that** the housing recess (35) is arranged in a partition (16) separating two adjacent compartments (17) of the housing member (2).

15. Surgical clip cartridge according to any one of the preceding claims, **characterised in that** the holding members (21) are arranged on the outside of the surgical clip (3), **in that** they are pivoted in, in their holding position, in the direction of the centre of the surgical clip (3) and enter the opening (12; 30, 31) of the limbs (7, 8) from the outside to the inside, while, in the release position, they are pivoted outwardly away from the surgical clip (3).

16. Surgical clip cartridge according to any one of claims 1 to 14, **characterised in that** the holding members (21) are arranged on the inside of the surgical clip (3) **in that**, in their holding position, they are pivoted out in the direction away from the centre of the surgical clip (3) and enter the opening (12; 30, 31) of the limbs (7, 8) from the inside to the outside, while, in the release position, they are pivoted inward toward the centre of the surgical clip (3).

## Revendications

1. Etui d'agrafes de ligature comprenant un corps de stockage (2) et, stockées dans celui-ci, plusieurs agrafes de ligature (3) de configuration en forme de C, qui présentent deux ailes (7, 8) reliées mutuellement par une branche de liaison (6), le corps de stockage (2) présentant plusieurs emplacements (17) destinés à recevoir chacun une agrafe de ligature (3), avec, dans chaque emplacement (17), une surface d'appui (19) qui est adaptée au contour de l'agrafe de ligature (3) et contre laquelle vient s'appliquer respectivement une agrafe de ligature (3) avec sa surface intérieure, l'étui comprenant par ailleurs des organes de retenue (21) agencés sur le corps de stockage (2), qui peuvent basculer d'une position de retenue dans une position de dégagement, et qui, dans la position de retenue, fixent l'agrafe de ligature (3) dans son emplacement (17), et, dans la position de dégagement, sont éloignées de l'agrafe de ligature (3),
**caractérisé en ce que** dans les ailes (7, 8) sont prévus des ouvertures de passage (12 ; 30, 31) dans lesquelles viennent s'engager les organes de retenue (21) dans la position de retenue, et **en ce que** l'ouverture de passage (12 ; 30, 31) dans l'aile (7, 8) de l'agrafe de ligature (3) est une fente s'étendant le long des ailes (7, 8).

2. Etui d'agrafes de ligature selon la revendication 1, **caractérisé en ce que** les organes de retenue (21) portent sur leur côté supérieur, une surface de rampe de glissement (24) pour un outil de prélèvement (25, 26), sur laquelle celui-ci vient appuyer lors de l'avancement le long des ailes (7, 8) d'une agrafe de ligature (3), en provoquant ainsi le basculement des organes de retenue (21) de la position de retenue dans la position de dégagement.

3. Etui d'agrafes de ligature selon l'une des revendications précédentes, **caractérisé en ce que** les organes de retenue (21) sont réalisés, dans leur zone (22) s'engageant dans l'ouverture de passage (12 ; 30, 31), en forme de barrette, et s'appuient, avec leurs parois latérales, contre les bords latéraux de l'ouverture de passage (12 ; 30, 31).

4. Etui d'agrafes de ligature selon l'une des revendications précédentes, **caractérisé en ce que** dans la branche de liaison (6) de l'agrafe de ligature (3) est agencée une ouverture de passage (12 ; 32) dans laquelle vient s'engager, lorsque l'agrafe de ligature (3) est appliquée sur la surface d'appui (19), une protubérance (20) de la surface d'appui (19).

5. Etui d'agrafes de ligature selon la revendication 4, **caractérisé en ce que** l'ouverture de passage (12 ; 32) dans la branche de liaison (6) de l'agrafe de ligature (3), est une fente s'étendant le long de la branche de liaison (6).

6. Etui d'agrafes de ligature selon la revendication 5, **caractérisé en ce que** la protubérance (20) est réalisée, dans sa zone s'engageant dans l'ouverture de passage (12 ; 32), en forme de barrette, et s'appuie, avec ses parois latérales, contre les bords latéraux de l'ouverture de passage (12 ; 32).

7. Etui d'agrafes de ligature selon l'une des revendications précédentes, **caractérisé en ce que** le corps de stockage (2) est réalisé d'un seul tenant avec les surfaces d'appui (19) et les organes de retenue (21).

8. Etui d'agrafes de ligature selon l'une des revendications précédentes, **caractérisé en ce que** le corps de stockage (2) est réalisé, avec les surfaces d'appui (19) et les organes de retenue (21), en matière plastique.

9. Etui d'agrafes de ligature selon la revendication 8, **caractérisé en ce que** les organes de retenue (21) sont reliés au corps de stockage (2) par l'intermédiaire d'une nervure de flexion (23), qui, lors du basculement des organes de retenue (21) de la position de retenue dans la position de dégagement, est déformée de manière plastique, permanente.

10. Etui d'agrafes de ligature selon l'une des revendications précédentes, **caractérisé en ce que** l'agrafe de ligature (3) présente deux nervures longitudinales (4, 5) s'étendant côte à côte et à distance l'une de l'autre, qui, aux extrémités des ailes (7, 8), sont reliées l'une à l'autre par une nervure transversale (13, 14), et définissent entre-elles un espace intermédiaire en forme de fente constituant les ouvertures de passage (12 ; 30, 31, 32) dans les ailes (7, 8) et, le cas échéant, dans la branche de liaison (6) de l'agrafe de ligature (3), ouvertures de passage dans lesquelles viennent s'engager les organes de retenue (21) et respectivement la protubérance (20) de la surface d'appui (19).

11. Etui d'agrafes de ligature selon la revendication 10, **caractérisé en ce que** les ouvertures de passage (30, 31) des ailes (7, 8) et l'ouverture de passage (32) dans la branche de liaison (6) de l'agrafe de ligature (3), sont séparées par des nervures transversales (28, 29) reliant les nervures longitudinales (4, 5) de l'agrafe de ligature (3).

12. Etui d'agrafes de ligature selon l'une des revendications précédentes, **caractérisé en ce que** les organes de retenue (21) sont fixés dans leur position de retenue, par rapport au corps de stockage (2), par une liaison par encliquetage élastique et amovible (33, 34, 35).

13. Etui d'agrafes de ligature selon la revendication 12, **caractérisé en ce que** l'organe de retenue (21) porte au moins une protubérance d'encliquetage latérale (33, 34), qui vient s'engager dans un évidement d'encliquetage (35) du corps de stockage (2).

14. Etui d'agrafes de ligature selon la revendication 13, **caractérisé en ce que** l'évidement d'encliquetage (35) est agencé dans une paroi de séparation (16), qui sépare deux emplacements (17) voisins du corps de stockage (2).

15. Etui d'agrafes de ligature selon l'une des revendications précédentes, **caractérisé en ce que** les organes de retenue (21) sont agencés du côté extérieur de l'agrafe de ligature (3), et **en ce que**, dans leur position de retenue, ils sont basculés vers l'intérieur en direction du centre de l'agrafe de ligature (3) et s'engagent de l'extérieur vers l'intérieur dans l'ouverture de passage (12 ; 30, 31) des ailes (7, 8), tandis que, dans la position de dégagement, ils sont basculés vers l'extérieur en s'éloignant de l'agrafe de ligature (3).

16. Etui d'agrafes de ligature selon l'une des revendications 1 à 14, **caractérisé en ce que** les organes de retenue (21) sont agencés du côté intérieur de l'agrafe de ligature (3), et **en ce que**, dans leur position de retenue, ils sont basculés vers l'extérieur en s'éloignant du centre de l'agrafe de ligature (3) et s'engagent de l'intérieur vers l'extérieur dans l'ouverture de passage (12 ; 30, 31) des ailes (7, 8), tandis que, dans la position de dégagement, ils sont basculés vers l'intérieur en direction du centre de l'agrafe de ligature (3).
